Europäisches Patentamt

European Patent Office

Office européen des brevets

(11) Publication number: **0 374 499 B1**

# EUROPEAN PATENT SPECIFICATION

(45) Date of publication of patent specification: **02.02.94**  (51) Int. Cl.5: **A01N 63/04**

(21) Application number: **89121529.5**

(22) Date of filing: **21.11.89**

(54) **Weed control compositions containing Drechslera spp. or a metabolite thereof and weed control methods using Drechslera spp. or a metabolite thereof.**

(30) Priority: **21.11.88 JP 292466/88**

(43) Date of publication of application:
**27.06.90 Bulletin  90/26**

(45) Publication of the grant of the patent:
**02.02.94 Bulletin  94/05**

(84) Designated Contracting States:
**CH DE FR GB IT LI NL**

(56) References cited:
**EP-A- 0 148 445
EP-A- 0 207 653
US-A- 4 606 751**

**AGRICULTURAL AND BIOLOGICAL CHEMISTRY, vol. 46, no. 11, 1982, pages 2681-2683, Agricultural Chemical Society of Japan, Tokyo, JP; D.J. ROBESON et al.: "Monocerin, a Phytotoxin from Exserohilum turcicum (= Drechslera turcica)"**

**PHYTOPARASITICA, vol. 16, no. 2, 1988, pages 145-152, Agricultural Research Organisation, The Volcani Center, Division of Scientific Publications, Bet Dagan, IL; G. STROBEL et al.: "The incredible fungal ge-**

**nus-Drechslera - and its phytotoxic ophiobolins"**

(73) Proprietor: **MITSUI TOATSU CHEMICALS, Inc.
2-5 Kasumigaseki 3-chome
Chiyoda-Ku Tokyo 100(JP)**

(72) Inventor: **Arita, Masanobu
1-4-1409-932, Shiomidai
Isogu-ku
Yokohama-shi Kanagawa-ken(JP)**
Inventor: **Yamaguchi, Kenichi
7-14-7, Fukuda
Yamato-shi Kanagawa-ken(JP)**

(74) Representative: **VOSSIUS & PARTNER
Postfach 86 07 67
D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 374 499 B1

## Description

The present invention relates to weed control compositions comprising living fungal cells or metabolites of plant pathogens effective for the control of weeds, especially, barnyard grass (*Echinochloa*) which is a troublesome weed in the cultivation of economic crops, for example, important crops of *Gramineae* such as rice and wheat, and also to methods for controlling weeds by using metabolites of such fungi.

Regarding control methods of weeds, plant diseases and insects, substantial developments have taken place in approximately the last 100 years owing to developments in chemistry. As a result, chemical control methods have replaced traditional cultivation control methods. In particular, the rapid progress of synthesized organic pesticides in the middle of the 19th century, typified by DDT, led to increased field crop yields, improved quality, farming labor saving, etc., so that the world food production was improved rapidly. Serious problems have, however, arisen in the last several years from the use of such synthesized organic pesticides, including environmental pollution and reduced control effects, resulting from the occurrence of pathogens and insect pests with acquired pesticide resistance. In recent years, the development of synthetic pesticides has therefore apparently been oriented towards a higher activity of the pesticides. Namely, pesticides are developed these days, which are harmless to mammals and display good effects at low application rates while paying sufficient attention to residuality, environmental pollution and the like. On the other hand, there has recently been a growing interest in so-called organic agriculture systems in which crops are cultivated without pesticides. People are very concerned with the influence of synthetic organic pesticides to the human body and their influence to the natural environment as mentioned above and in recent years, the interests in biopesticides has increased enormously, so that a great deal of research and development work is under way with respect to biopesticides. The establishment of a total control system relying upon the combination of a biological control method, which makes use of a natural enemy or the like, and a cultivation or cultural control method involving the use of a cultivation system, such as crop rotation, may be mentioned by way of example.

From the foregoing background, there is a keen demand for the establishment of biological control methods. Great hope is placed especially on the development of biopesticides directly using the living cells of a microorganism and microbial origin pesticides using a physiologically active substance produced by a microorganism. In the field of herbicides, extensive investigations have been carried out in recent years on mycoherbicides which use a pathogen against weeds. Mycoherbicides which have already been put on the market include "DeVine", (trade mark; product of Abbott Laboratories Ltd.) and "Collego" (trade mark; product of Ecogen Ltd.). These two mycoherbicides are employed for the control of strangle vine and northern jointvetch, respectively. In addition, a mycoherbicide effective for the control of sicklepod in peanut and soybean fields is said to be commercially available soon from Mycogen Ltd. Neither mycoherbicide nor microbial origin herbicides are yet available against barnyard grass (*Echinochloa spp.*) which is a troublesome weed in the cultivation of important crops such as rice and wheat.

An object of this invention is to solve the above-described current deficiencies in the production of the crops and hence to provide novel weed control means capable of taking over the position of synthesized herbicides.

The present inventors have found certain new strains of the fungus *Drechslera spp.* as pathogens effective for the control of barnyard grass (*Echinochloa spp.*) which is a troublesome weed in the cultivation of economic crops such as rice and wheat.

The present invention therefore provides a weed control composition comprising living *Drechslera spp.* fungal cells or a metabolite thereof as well as a weed control method using living fungal cells or a metabolite of *Drechslera spp.*.

The pathogens, *Drechslera spp.*, useful in the practice of this invention exhibit a specific pathogenecity to barnyard grass. The use of these microorganisms as mycoherbicide makes it possible to control barnyard grass, a troublesome weed, without adversely affecting adjacent economic crops.

Further, the pathogens which are useful in the practice of this invention have been chosen from a variety of naturally occurring microorganisms and therefore are free of the potential problem of environmental pollution by synthesized organic herbicides and can be used safely.

With a view toward controlling barnyard grass which is a troublesome weed in both upland and paddy fields, the present inventors were interested in pathogens against barnyard grass and therefore investigated numerous microorganisms of this type. As a result, many of the mold fungi *Drechslera spp.* are pathogens which possess herbicidal activities against barnyard grass but are non-pathogenic to crops such as rice and wheat, leading to the completion of this invention.

Barnyard grass, which is the target weed of the present invention, is a plant belonging to the genus of *Echinochloa* which in turn falls under the order of *Graminales* and specifically includes *Echinochloa*

2

*oryzicola, Echinochloa crus-galli var. formosensis, Echinochloa crus−galli va. crus−galli*, and *Echinochloa crus−galli var. praticola.*

Namely, *Drechslera spp.* useful in the practice of this invention were found by subjecting to pure isolation pathogens, which had been collected from lesions of naturally-infected barnyard grass, and then selecting, from the thus-isolated pathogens, those being pathogenic to barnyard grass but non-pathogenic to economic crops typified by rice.

Pathogens useful in the practice of this invention were selected by conducting both herbicidal activity and parasitic activity tests on barnyard grass and rice plants with respect to strains isolated from naturally-infected barnyard grass. As a result of a morphological identification of the obtained six strains with the activity of the controlling barnyard grass, they were found to be classified as *Drechslera monoceras* [3 strains; MH-0015 (FERM P-10785), MH-2653 (FERM P-10786), MH-2679 (FERM P-10982)], *Drechslera ravenellii* [2 strains; MH-0042 (FERM P-10367), MH-0060 (FERM P-11033)], and *Drechslera poa* [1 strain; MH-0122 (FERN P-10981)]. These depositions were converted to depositions under the requirements of the Budapest Treaty. The corresponding new deposition numbers are FERM BP-2652, FERM BP-2653, FERM BP-2656, FERM BP-2659, FERM BP-2657 and FERM BP-2655, respectively. The depositions were effected at the Fermentation Research Institute, Agency of Industrial Science and Technology, Japan, on June 17, 1989; June 17, 1989; August 31, 1989; October 28, 1989; September 28, 1989; and August 31, 1989, respectively.

The weed control compositions of this invention, which possess the specific pathogenecity against barnyard grass only, can use any one of the above-selected strains of *Drechslera spp.*.

As a method for using *Drechslera spp.* in such weed control compositions, cultured living fungal cells can be used directly as they are, or after culturing the cells, the culture resulting from germ-free filtration to use a metabolite thereof.

According to the former method, a weed control composition can be obtained by suspending spores, which have been obtained by culturing *Drechslera spp.* on a nutrient medium, in an aqueous solution of a non-ionic surfactant such as "Triton X-100" (trade name) or "Tween 80" (trade name)

Spores of Drechslera spp. can be obtained regardless of whether the nutrient medium is in a liquid form or in a solid form. When a liquid culture medium is desired, a potato-dextrose liquid culture medium or an oatmeal liquid culture medium is inoculated with cells. The cells are allowed to grow there. Resultant cells are collected. Mycelia of *Drechslera spp.* thus obtained are ground, spread on a filter paper and then left to grow and form spores. When a solid culture medium is desired on the other hand, cells are cultured on a potato-dextrose agar medium or oatmeal agar medium. Aerial hyphae are then removed with distilled water whereby spores of *Drechslera spp.* can be obtained.

On the other hand, according to the latter method in which a cell metabolite is used, *Drechslera spp.* are subjected to static culture on a potato-dextrose liquid culture medium. The resulting culture is subjected to germ-free filtration and the filtrate containing the cellular metabolites is formulated into a weed control composition.

Effective microbial weed control compositions and microbial origin weed control compositions can be produced by mass-culturing *Drechslera spp.* and efficiently obtaining spores, both under asceptic conditions. When applied to the field, i.e., paddy fields before and after transplantation of rice seedlings, these weed control compositions possess selective herbicidal activities against barnyard grass only and show substantially no pathogenicity against economic crops such as rice, wheat, barley and corn. They therefore have highly-selective herbicidal activities, and are free of the potential problem of environmental contamination and thus can be used safely.

Pathogens *Drechslera spp.* useful in the practice of this invention will hereinafter be specifically described by the following examples. It should however be borne in mind that the present invention is not limited thereto.

Example 1:

Isolation and Identification of Effective Pathogens

1) Isolation method of pathogens:

Naturally-infected barnyard grass was collected from a paddy field. Centering at individual lesions, leaf pieces of 10-20 mm long were cut off. The pieces of barnyard grass were dipped for 1-2 seconds in a 70% ethyl alcohol solution and then for 10 minutes in sodium hypochlorite solution having an effective chlorine concentration of 2%, whereby the pieces of barnyard grass were subjected to surface sterilization. The surfac-sterilized pieces of barnyard grass were washed three times with distilled water and then placed on a plate with nutrient-free agar medium. The static cultivation was carried out at 25°C for 72 hours. Hyphae of mold fungi thus grown were subjected to single hyphae isolation under a microscope, followed by purification on a nutrient medium. Using the microorganism thus isolated, its herbicidal activities and parasiticity against barnyard grass and its pathogenecity against rice were tested.

2) Test for herbicidal activities of isolated microorganisms against barnyard grass and rice plant:

Barnyard grass and rice were allowed to grow to the 1.5 leaf stage in test tubes to provide test samples. Namely, barnyard grass seeds and rice seeds were dipped for 1-2 seconds in a 70% ethyl alcohol solution and then for 10 minutes in sodium hypochlorite solution having an effective chlorine concentration of 2%, whereby the seeds were subjected to surface sterilization. The seeds were then washed three times with distilled water. The sterilized seeds were then planted in test tubes which contained B5 agar medium of the below-described composition and were allowed to grow in a plant growth chamber.

## Composition of B5 Medium

| Macroelement | mg/ℓ | mM | Microelement | mg/ℓ | mM |
|---|---|---|---|---|---|
| $KNO_3$ | 2500 | 25 | KI | 0.75 | 4.5 |
| $CaCl_2 \cdot 2H_2O$ | 150 | 1.0 | $H_3BO_3$ | 3.0 | 50 |
| $MgSO_4 \cdot 7H_2O$ | 250 | 1.0 | $MnSO_4 \cdot H_2O$ | 10 | 60 |
| $(NH_4)_2SO_4$ | 134 | 1.0 | $ZnSO_4 \cdot 7H_2O$ | 2.0 | 7.0 |
| $NaH_2PO_4 \cdot H_2O$ | 150 | 1.1 | $Na_2MoO_4 \cdot 2H_2O$ | 0.25 | 1.0 |
| Vitamin | mg/ℓ | | $CuSO_4 \cdot 5H_2O$ | 0.025 | 0.1 |
| Inositol | 100 | | $CoCl_2 \cdot 6H_2O$ | 0.025 | 0.1 |
| Nicotinic acid | 1.0 | | $Na_2 \cdot EDTA$ | 37.3 | 100 |
| Pyridoxine | 1.0 | | $FeSO_4 \cdot 7H_2O$ | 27.8 | 100 |
| Thiamine | 10.0 | | Sugar | 20 g/ℓ | |
| | | | pH | 5.5 | |

On the other hand, the isolated microorganisms were subjected to plate culture on separate layers of a potato-dextrose agar culture medium, respectively. The layers were punched out by a sterilized cork borer to obtain mycelial discs as seed cell sources.

The mycelial discs were separately placed on the culture medium in the test tubes in which barnyard grass seedlings and rice seedlings were grown aseptically. After incubating them for 10 days in a plant growth chamber, the herbicidal activities of the microorganisms were tested. The results are shown in Table 1.

4

Table 1

| Herbicidal Activities of Isolated Microorganisms against Barnyard Grass and Rice | | |
|---|---|---|
| Microorganism | Barnyard grass | Rice |
| MH-0001 | - | - |
| MH-0007 | - | + + + |
| MH-0015 (FERM P-10785) | + + + | - |
| MH-0023 | + + + | + + + |
| MH-0042 (FERM P-10367) | + + + | - |
| MH-0060 (FERM P-11033) | + + + | - |
| MH-0122 (FERM P-10981) | + + + | - |
| MH-2653 (FERM P-10786) | + + + | - |
| MH-2679 (FERM P-10982) | + + + | - |
| Note:<br>+ + + Death<br>+ + Marked inhibition to the growth<br>+ Some inhibition to the growth<br>- No effect | | |

3) Test for the parasitic activity of isolated microorganisms on barnyard grass and rice:

Barnyard grass and rice were allowed to grow to the 1.5 leaf stage in test tubes to provide test samples. Namely, barnyard grass seeds and rice seeds were subjected to surface sterilization in a manner similar to the above herbicidal activity test 2). They were planted in test tubes which contained a nutrient-free liquid medium sterilized in advance, followed by culture in a plant growth chamber. On the other hand, from the isolated microorganisms, mycelial discs were prepared as inoculation sources in a manner similar to the above herbicidal activity test 2).

The target plants were inoculated with the microbial inoculation sources and then cultured for 10 days. Then, the parasiticity of each microorganism was determined. The results are summarized in Table 2.

5

Table 2

| Parasiticity of Isolated Microorganisms on Barnyard Grass and Rice | | |
|---|---|---|
| Microorganism | Barnyard grass | Rice |
| MH-0001 | - | - |
| MH-0007 | - | + + + |
| MH-0015 (FERM P-10785) | + + + | - |
| MH-0023 | - | - |
| MH-0042 (FERM P-10367) | + + + | - |
| MH-0060 (FERM P-11033) | + + | - |
| MH-0122 (FERM P-10981) | + + | - |
| MH-2653 (FERM P-10786) | + + + | - |
| MH-2679 (FERM P-10982) | + + + | - |
| Note:<br>+ + + Death<br>+ + Marked inhibition to the growth<br>+ Some inhibition to the growth<br>- No effect | | |

As shown in Table 1, the microorganisms useful in the practice of this invention [*Drechslera spp.* MH-0015 (FERM P-10785), MH-0042 (FERM P-10367), MH-0060 (FERM P-11033), MH-0122 (FERM P-10981), MH-2653 (FERM P-10786) and MH-2679 (FERM P-10982)] have high herbicidal activities against barnyard grass. The various isolated microorganisms included strains incapable of showing pathogenecity against barnyard grass such as MH-0001, strains capable of showing pathogenecity against rice only such as MH-0007 and strains capable of exhibiting high pathogenecity against both barnyard grass and rice. From these numerous microorganisms, the strains useful in the practice of this invention have been selected. Further, the strains useful in the practice of this invention, which are shown in Table 2, have specific parasiticity to barnyard grass, so that they are very advantageous when employed as mycoherbicides.

4) Identification of isolated microorganisms:

Identification was conducted with respect to the 6 strains which exhibited excellent herbicidal activities and parasiticity against barnyard grass but were not recognized to have effects on grass in the tests 2) and 3), namely, MH-0015 (FERM P-10785), MH-0042 (FERM P-10367), MH-0060 (FERM P-11033), MH-0122 (FERM P-10981), MH-2653 (FERM P-10786) and MH-2679 (TERM P-10982). The identification of these strains was effected by culturing the microorganisms with malt-agar medium. As a result, MH-0015 (FERM P-10785), MH-2653 (FERM P-10786) and MH-2679 (FERM P-10982) gave a colony as large as 65-75 mm in diameter in 7 days at 28°C and showed irregular growth. These colonies had a grayish black color. Conidia had scars and their sizes were 15-17.5 $\mu$m in width and 87.5-127.5 $\mu$m in length. They had a somewhat bent shape. The conidia had 9 septa at the maximum and often, 5-7 septa. Conidiophores had straight shapes. From the above characteristics, these three types of microorganisms were identified to be strains of *Drechslera monoceras*.

MH-0042 (FERM P-10367) and MH-0060 (FERM P-11033) had conidia which contained no scars and had a size of 17-22 $\mu$m in width and 40-90 $\mu$m in length. The conidia were often branched. Regarding the shape of the conidia, some bent cylindrical conidia were observed. They contained 1-5 septa. From the foregoing characteristics, these two types of microorganisms were identified to be strains of *Drechslera ravenellii*.

MH-0122 (FERM P-10981) gave a colony as large as 20-25 mm in 7 days at 28°C. The colony had a bright gray color, but was grayish green in the central part thereof and grayish black on the back. Its conidia were free of scars and had a size of 20-25 $\mu$m in width and 55-95 $\mu$m in length. The conidia contained 5-6 septa. Conidiophores had a straight shape. From the above characteristics, this microorganism was found to be a strain of *Drechslera poa*.

These determinations were conducted with reference to M.B. Ellis, "Demariaceus Hyphomycetes", 608, Commonwealth Mycological Institute, Kew, England (1971) and M.B. Ellis, "More Demariaceus Hyphomycetes", 507, Commonwealth Mycological Institute, Kew, England (1976).

The *Drechslera spp.* useful in the practice of this invention are not described as pathogens in the Pathogens Safety Control Guideline compiled by the National Institutes of Health (Japan) and are believed to be safe to mammals.

Example 2:

Formulation Method of Weed Control Compositions Containing living fungal cells of *Drechslera Spp.* and Weed Control Method Using the Compositions

The 6 strains of *Drechslera spp.* isolated above were separately inoculated on an oatmeal-agar medium and then subjected to static culture at 25°C for 7 days. Aerial hyphae were then removed with distilled water, thereby obtaining spores. They were separately suspended at a concentration of $10^6$ spores/ml in a 0.05% "Triton X-100" (trade name) solution, whereby weed control compositions containing, as an effective ingredient, living fungal cells of the respective strains of *Drechslera spp.* were formulated.

On the other hand, seeds of barnyard grass and rice were planted in soil contained in PVC pots having a diameter of 10 cm and were reared to seedlings of the 1.5 leaf stage, respectively. The weed control compositions were separately poured dropwise into the pots under irrigation so as to inoculate $10^7$ spores of the strains of *Drechslera spp.* per pot. The pots were maintained at 30°C during the daytime and at 25°C at night. Rearing was conducted for 20 days in a weather-controlled room. The results are shown in Table 3.

Table 3

| Selective Herbicidal Effects of Weed Control Compositions Containing Living Fungal Cells of *Drechslera spp.* | | |
|---|---|---|
| Microorganism | Barnyard grass | Rice |
| MH-0015 (FERM P-10785) | + + + | - |
| MH-0042 (FERM P-10367) | + + + | - |
| MH-0060 (FERM P-11033) | + + + | - |
| MH-0122 (FERM P-10981) | + + + | - |
| MH-2653 (FERM P-10786) | + + + | - |
| MH-2679 (FERM P-10982) | + + + | - |
| Note:<br>+ + + Death<br>+ + Marked inhibition to the growth<br>+ Some inhibition to the growth<br>- No effect | | |

As a result of the test, the pathogens useful in the practice of this invention, namely, *Drechslera spp.* MH-0015 (FERM P-10785), MH-0042 (FERM P-10367), MH-0060 (FERM P-11033), MH -0122 (FERM P-10981), MH-2653 (FERM P-10786) and MH-2679 (FERM P-10982) showed high pathogenecity against barnyard grass and effects to rice seedlings were not observed at all.

Example 3:

Formulation Method of Weed Control Composition Containing as Effective Ingredient *Drechslera monoceras* MH-2653 (FERM P-10786) and Weed Control Method Using the Composition

Inoculated on 100 ml of a potato-dextrose liquid culture medium was a seed culture of the pathogen useful in the practice of this invention, *Drechslera monoceras* MH-2653 (FERM P-10786), followed by culture at 25°C for 7 days. After the culture, the obtained mycelia were homogenized in culture medium, and spread on a filter paper and then allowed to stand for 3 days to form spores. Using these spores, a weed control composition was formulated in a similar manner to Example 2.

On the other hand, 50 barnyard grass seeds were planted in each of three 1/5000-a Wagner pots and were allowed to grow to the 1.5 leaf stage. Into the pots under irrigation, the weed control composition was applied dropwise so as to inoculate the pots with $10^5$, $10^6$, $10^7$ and $10^8$ spores of *Drechlera monoceras* MH-2653 (FERM P-10786),respectively. They were reared in a green house whose temperature was controlled within a range of from 15°C to 35°C. Herbicidal activities were determined based on the percentage of dead seedlings.

As a result of the test, *Drechslera monoceras* MH-2653 (FERM P-10786) useful in the practice of this invention shows excellent herbicidal activities against barnyard grass.

Table 4

| Effects of spore dosage on herbicidal activity of *Drechslera spp.* MH-2653 (FERM P-10786) | |
|---|---|
| Inoculum size (spores/pot) | Control value (%) |
| 0 | 0 |
| $1 \times 10^5$ | 54 |
| $1 \times 10^6$ | 86 |
| $1 \times 10^7$ | 100 |
| $1 \times 10^8$ | 100 |

Example 4:

Formulation Method of Weed Control Composition Containing A Metabolite of *Drechslera spp.* and Weed Control Method Using the Composition

The isolated 6 strains of *Drechslera spp.* were separately subjected to standing culture at 25°C for 10 days, each, on 100 mℓ of a potato-dextrose liquid culture medium. Thereafter, mat-like mycelium samples thus obtained were separately homogenized in a liquid culture medium. The resulting culture broths were separately filtered through a membrane filter and the filtrates were separately concentrated for use as weed control compositions.

On the other hand, barnyard grass seeds were subjected to surface sterilization in a manner similar to the herbicidal activity test in Example 1 and then planted in portions in test tubes which contained 5 mℓ of a nutrient-free liquid culture medium. They were reared until the 1.5 leaf stage in a plant-growth chamber. The liquid weed control compositions were separately poured at a rate of 0.5 mℓ into the test tubes with the barnyard grass seedlings reared therein. The seedlings were reared for 10 days, and the herbicidal activities of the individual compositions were determined. The results are shown in Table 5.

Table 5

| Herbicidal Activities of Weed Control Compositions Containing Metabolite of *Drechslera spp.* against Barnyard Grass | |
|---|---|
| Microorganism | Herbicidal activities |
| MH-0001 | - |
| MH-0007 | - |
| MH-0015 (FERM P-10785) | + + + |
| MH-0042 (FERM P-10367) | + + + |
| MH-0060 (FERM P-11033) | + + |
| MH-0122 (FERM P-10981) | + + |
| MH-2653 (FERM P-10786) | + + + |
| MH-2679 (FERM P-10982) | + + + |
| Note:<br>+ + + Death<br>+ + Marked inhibition to the growth<br>+ Some inhibition to the growth<br>- No effect | |

As a result of the test, the metabolites of *Drechslera spp.* MH-0015 (FERM P-10785), MH-0042 (FERM P-10367), MH-0060 (FERM P-11033), MH-0122 (FERM P-10981), MH-2653 (FERM P-10786) and MH-2679 (FERM P-10982) showed marked herbicidal activities against barnyard grass.

Example 5:

Pathogenecity Test of *Drechslera Spp.* for Primary Crops

The pathogens *Drechslera spp.* useful in the practice of this invention were found to have no pathogenecity against economic crops. In a manner similar to Example 3, the test was conducted using rice, wheat, barley and corn as target plants. The results are shown in Table 6.

Table 6

| Pathogenecity of *Drechslera spp.* against Primary Crops | | | | |
|---|---|---|---|---|
| Microorganism | Rice | Wheat | Barley | Corn |
| MH-0015 (FERM P-10785) | - | - | - | - |
| MH-0042 (FERM P-10367) | - | - | - | - |
| MH-0060 (FERM P-11033) | - | - | - | - |
| MH-0122 (FERM P-10981) | - | - | - | - |
| MH-2653 (FERM P-10786) | - | - | - | - |
| MH-2679 (FERM P-10982) | - | - | - | - |
| Note:<br>+ + + Death<br>+ + Marked inhibition to the growth<br>+ Some inhibition to the growth<br>- No effect | | | | |

As a result of the test, *Drechslera spp.* useful in the practice of this invention were found to have absolutely no pathogenecity against rice, wheat, barley and corn. These microorganisms of this invention were hence recognized to be usable as mycoherbicides.

## Claims

1. A weed control composition comprising an agriculturally acceptable carrier or diluent and *Drechslera spp.* which is selected from the group consisting of *Drechslera monoceras*, *Drechslera ravenellii* or *Drechslera poa*, which are non-pathogenic to economic crops but pathogenic to *Echinochloa spp.*

2. The composition of claim 1, wherein the composition is in the form of a solution containing living fungal cells of *Drechslera spp.* suspended at a concentration of $10^4$-$10^9$ cells per milliliter of the solution.

3. The composition of claim 1 or 2, wherein spores of *Drechslera spp.* are used.

4. A method for controlling weeds which comprises applying to a locus a weed control composition of claim 1.

5. A method according to claim 4, wherein spores of *Drechslera spp.* of claim 1 are applied.

6. A metabolite of *Drechslera monoceras*, *Drechslera ravenellii* or *Drechslera poa* of *Drechslera spp.* being pathogenic to *Echinochloa spp.* and being obtainable by subjecting said *Drechslera* species to a standing culture in potato-dextrose liquid culture medium, homogenizing the resulting mycelium samples in a liquid medium through a membrane filter and concentrating the filtrates for use in a weed control composition.

7. A weed control composition comprising a metabolite according to claim 6 and an agriculturally acceptable carrier or diluent.

8. A method for controlling weeds which comprises applying to a locus a metabolite according to claim 6.

9. The composition, method or metabolite of any one of claims 1 to 8, wherein *Drechslera spp.* is a strain of *Drechslera monoceras*.

10. The composition, method or metabolite of claim 9, wherein the strain is MH-0015 (FERM P-10785).

11. The composition, method or metabolite of claim 9, wherein the strain is MH-2653 (FERM P-10786).

12. The composition, method or metabolite of claim 9, wherein the strain is MH-2679 (FERM P-10982).

13. The composition, method or metabolite of any one of claims 1 to 8, wherein *Drechslera spp.* is a strain of *Drechslera ravenellii*.

14. The composition, method or metabolite of claim 13, wherein the strain is MH-0042 (FERM P-10367).

15. The composition, method or metabolite of claim 13, wherein the strain is MH-0060 (FERM P-11033).

16. The composition, method or metabolite of any one of claims 1 to 8, wherein *Drechslera spp.* is a strain of *Drechslera poa*.

17. The composition, method or metabolite of claim 16, wherein the strain is MH-0122 (FERM P-10981).

18. A strain of *Drechslera spp*, which is the strain MH-0015 (FERM P-10785), MH-2653 (FERM P-10786), MH-2679 (FERM P-10982), MH-0042 (FERM P-10367), MH-0060 (FERM P-11033), or MH-0122 (FERM P-10981).

19. Spores of a strain of *Drechslera spp.* of claim 18.

**Patentansprüche**

1. Eine Zusammensetzung zur Unkrautbekämpfung, umfassend einen landwirtschaftlich verträglichen Träger oder ein landwirtschaftlich verträgliches Verdünnungsmittel und Drechslera spp., welches ausgewählt ist aus der Gruppe bestehend aus Drechslera monoceras, Drechslera ravenellii oder Drechslera poa, die auf wirtschaftlich bedeutsame Getreide nicht-pathogen aber pathogen auf Echinochloa spp. wirken.

2. Die Zusammensetzung nach Anspruch 1, wobei die Zusammensetzung in Form einer Lösung vorliegt, die lebende Drechslera spp. Pilzzellen enthält, die in einer Konzentration von $10^4$-$10^9$ Zellen pro ml der Lösung suspendiert sind.

3. Die Zusammensetzung nach Anspruch 1 oder 2, wobei Sporen von Drechslera spp. verwendet werden.

4. Ein Verfahren zur Bekämpfung von Unkräutern, umfassend die Anwendung einer Zusammensetzung zur Unkrautbekämpfung nach Anspruch 1 auf einen Ort.

5. Ein Verfahren nach Anspruch 4, wobei Sporen von Drechslera spp. nach Anspruch 1 verwendet werden.

6. Ein Metabolit von Drechslera monoceras, Drechslera ravenellii oder Drechslera poa aus Drechslera spp., der auf Echinochloa spp. pathogen wirkt und erhältlich ist durch Kultivieren der Drechslera-Arten in einer Standkultur in flüssigem Kartoffel-Dextrose-Kulturmedium, Homogenisieren der erhaltenen Mycelproben mittels eines Membranfilters in einem flüssigen Medium und Konzentrieren der Filtrate zur Verwendung in einer Zusammensetzung zur Unkrautbekämpfung.

7. Eine Zusammensetzung zur Unkrautbekämpfung, umfassend einen Metaboliten nach Anspruch 6 und einen landwirtschaftlich verträglichen Träger oder ein landwirtschaftlich verträgliches Verdünnungsmittel.

8. Ein Verfahren zur Bekämpfung von Unkräutern, umfassend die Anwendung eines Metaboliten nach Anspruch 6 auf einen Ort.

9. Die Zusammensetzung, das Verfahren oder der Metabolit nach einem der Ansprüche 1 bis 8, wobei Drechslera spp. ein Stamm von Drechslera monoceras ist.

10. Die Zusammensetzung, das Verfahren oder der Metabolit nach Anspruch 9, wobei der Stamm MH-0015 (FERM P-10785) ist.

11. Die Zusammensetzung, das Verfahren oder der Metabolit nach Anspruch 9, wobei der Stamm MH-2653 (FERM P-10786) ist.

12. Die Zusammensetzung, das Verfahren oder der Metabolit nach Anspruch 9, wobei der Stamm MH-2679 (FERM P-10982) ist.

13. Die Zusammensetzung, das Verfahren oder der Metabolit nach einem der Ansprüche 1 bis 8, wobei Drechslera spp. ein Stamm von Drechslera ravenellii ist.

14. Die Zusammensetzung, das Verfahren oder der Metabolit nach Anspruch 13, wobei der Stamm MH-0042 (FERM P-10367) ist.

15. Die Zusammensetzung, das Verfahren oder der Metabolit nach Anspruch 13, wobei der Stamm MH-0060 (FERM P-11033) ist.

16. Die Zusammensetzung, das Verfahren oder der Metabolit nach einem der Ansprüche 1 bis 8, wobei Drechslera spp. ein Stamm von Drechslera poa ist.

**17.** Die Zusammensetzung, das Verfahren oder der Metabolit nach Anspruch 16, wobei der Stamm MH-0122 (FERM P-10981) ist.

**18.** Ein Stamm von Drechslera spp, der der Stamm MH-0015 (FERM P-10785), MH-2653 (FERM P-10786), MH-2679 (FERM P-10982), MH-0042 (FERM P-10367); MH-0060 (FERM P-11033) oder MH-0122 (FERM P-10981) ist.

**19.** Sporen eines Stammes von Drechslera spp. nach Anspruch 18.

**Revendications**

**1.** Composition herbicide comprenant un support ou diluant acceptable en agriculture et *Drechslera spp.* qui est choisie dans le groupe constitué par *Drechslera monoceras*, *Drechslera ravenellii* ou *Drechslera poa*, qui sont non pathogènes pour les cultures économiques mais pathogènes pour *Echinochloa spp*.

**2.** Composition selon la revendication 1, dans laquelle la composition est sous forme d'une solution contenant des cellules fongiques vivantes de *Drechslera spp.* en suspension à une concentration de $10^4$-$10^9$ cellules par millilitre de solution.

**3.** Composition selon la revendication 1 ou 2, dans laquelle on utilise des spores de *Drechslera spp*.

**4.** Méthode de lutte contre les mauvaises herbes qui comprend l'application dans un lieu de la composition herbicide de la revendication 1.

**5.** Méthode selon la revendication 4, dans laquelle on applique des spores de *Drechslera spp*. de la revendication 1.

**6.** Métabolite de *Drechslera monoceras*, *Drechslera ravenellii* ou *Drechslera poa* de *Drechslera spp*., pathogène pour *Echinochloa spp*. et pouvant être obtenu par soumission de ladite espèce de *Drechslera* à une culture stationnaire dans un milieu de culture liquide de pomme de terre-dextrose, homogénéisation des échantillons de mycélium obtenus dans un milieu liquide à travers un filtre à membrane et concentration des filtrats, pour l'utilisation dans une composition herbicide.

**7.** Composition herbicide comprenant un métabolite selon la revendication 6 et un support ou diluant acceptable en agriculture.

**8.** Méthode de lutte contre les mauvaises herbes, qui comprend l'application dans un lieu d'un métabolite selon la revendication 6.

**9.** Composition, méthode ou métabolite selon l'une quelconque des revendications 1 à 8, où *Drechslera spp*. est une souche de *Drechslera monoceras*.

**10.** Composition, méthode ou métabolite selon la revendication 9, où la souche est MH-0015 (FERM P-10785).

**11.** Composition, méthode ou métabolite selon la revendication 9, où la souche est MH-2653 (FERM P-10786).

**12.** Composition, méthode ou métabolite selon la revendication 9, où la souche est MH-2679 (FERM P-10982).

**13.** Composition, méthode ou métabolite selon l'une quelconque des revendications 1 à 8, où *Drechslera spp*. est une souche de *Drechslera ravenellii*.

**14.** Composition, méthode ou métabolite selon la revendication 13, où la souche est MH-0042 (FERM P-10367).

**15.** Composition, méthode ou métabolite selon la revendication 13, où la souche est MH-0060 (FERM P-11033).

**16.** Composition, méthode ou métabolite selon l'une quelconque des revendications 1 à 8, où *Drechslera spp.* est une souche de *Drechslera poa.*

**17.** Composition, méthode ou métabolite selon la revendication 16, où la souche est MH-0122 (FERM P-10981).

**18.** Souche de *Drechslera spp.*, qui est la souche MH-0015 (FERM P-10785), MH-2653 (FERM P-10786), MH-2679 (FERM P-10982), MH-0042 (FERM P-10367), MH-0060 (FERM P-11033), ou MH-0122 (FERM P-10981).

**19.** Spores d'une souche de *Drechslera spp.* de la revendication 18.